# EUROPEAN PATENT APPLICATION

(11) **EP 1 580 281 A2**
(43) Date of publication of application: **28.09.2005**
(21) Application number: 04019288.2
(22) Date of filing: 13.08.2004
(51) Int. Cl.: C12Q 1/68

(54) **Method for microbial antibiotic resistance detection**

(30) Priority: 29.09.2003 US 673038
(71) Applicant: EPPENDORF AG, 22339 Hamburg (DE)
(72) Inventor: Jung, Verena, 70736 Fellbach (DE); Susa, Milorad, 70376 Stuttgart (DE); Schmidt, Rolf, 70329 Stuttgart (DE); Ezaki, Satoshi, Ibaragi 301-0044 (JP); Knabbe, Cornelius, 70193 Stuttgart (DE); Bachmann, Till T., 70569 Stuttgart (DE)
(74) Representative: Becker Kurig Straus

(57) **Abstract**

The present invention pertains to a method for a rapid detection of microbial beta-lactam resistance using a micro-array and/or for a rapid detection of microbial beta-lactamase encoding nucleotide sequences and to a kit therefore.

### Annex to the application documents - subsequently filed sequences listing

SEQUENCE LISTING

<110> Eppendorf AG

<120> Method for microbial antibiotic resistance detection

<130> 80432 EP

<140> EP 04019288.2
<141> 2004-08-13

<150> US 10/673,038
<151> 2003-09-29

<160> 47

<170> PatentIn version 3.1

<210> 1
<211> 18
<212> DNA
<213> Artificial Sequence

<220>
<223> probe sequence

<400> 1
agaaacgctg gtgaaagt 18

<210> 2
<211> 18
<212> DNA
<213> Artificial Sequence

<220>
<223> probe sequence

<400> 2
tctagacagc cactcata 18

<210> 3
<211> 19
<212> DNA
<213> Artificial Sequence

<220>
<223> probe sequence

<400> 3
gattggacga gtcaggagc 19

<210> 4
<211> 18
<212> DNA
<213> Artificial Sequence

<220>
<223> probe sequence

<400> 4

tctagacagc cactcata                                                    18

<210>  5
<211>  22
<212>  DNA
<213>  Artificial Sequence

<220>
<223>  probe sequence

<220>
<221>  misc_feature
<222>  (10)..(10)
<223>  n = a, g, c or t

<400>  5
atgagtattn aacatttccg tg                                               22

<210>  6
<211>  20
<212>  DNA
<213>  Artificial Sequence

<220>
<223>  probe sequence

<220>
<221>  misc_feature
<222>  (10)..(10)
<223>  n = a, g, c or t

<400>  6
gcattttgcn ttcctgtttt                                                  20

<210>  7
<211>  19
<212>  DNA
<213>  Artificial Sequence

<220>
<223>  probe sequence

<220>
<221>  misc_feature
<222>  (9)..(9)
<223>  n = a, g, c or t

<400>  7
ctgaagatna gttgggtgc                                                   19

<210>  8
<211>  21
<212>  DNA
<213>  Artificial Sequence

<220>

<223>  probe sequence

<220>
<221>  misc_feature
<222>  (11)..(11)
<223>  n = a, g, c or t


<400>  8
cagttgggtg nacgagtggg t                                                21


<210>  9
<211>  27
<212>  DNA
<213>  Artificial Sequence

<220>
<223>  probe sequence

<220>
<221>  misc_feature
<222>  (14)..(14)
<223>  n = a, g, c or t


<400>  9
atcgaactgg atcncaacag cggtaag                                          27


<210>  10
<211>  26
<212>  DNA
<213>  Artificial Sequence

<220>
<223>  probe sequence

<220>
<221>  misc_feature
<222>  (13)..(13)
<223>  n = a, g, c or t


<400>  10
cgttttccaa tgntgagcac ttttaa                                           26


<210>  11
<211>  24
<212>  DNA
<213>  Artificial Sequence

<220>
<223>  probe sequence

<220>
<221>  misc_feature
<222>  (13)..(13)
<223>  n = a, g, c or t


<400>  11

ttttccaatg atnagcactt ttaa                                                24

<210>  12
<211>  20
<212>  DNA
<213>  Artificial Sequence

<220>
<223>  probe sequence

<220>
<221>  misc_feature
<222>  (12)..(12)
<223>  n = a, g, c or t

<400>  12
atgtggtgcg gnattatccc                                                20

<210>  13
<211>  19
<212>  DNA
<213>  Artificial Sequence

<220>
<223>  probe sequence

<220>
<221>  misc_feature
<222>  (10)..(10)
<223>  n = a, g, c or t

<400>  13
ttatcccgtn ttgacgccg                                                19

<210>  14
<211>  17
<212>  DNA
<213>  Artificial Sequence

<220>
<223>  probe sequence

<220>
<221>  misc_feature
<222>  (9)..(9)
<223>  n = a, g, c or t

<400>  14
gcaactcgnt cgccgca                                                17

<210>  15
<211>  20
<212>  DNA
<213>  Artificial Sequence

<220>

<223>  probe sequence

<220>
<221>  misc_feature
<222>  (10)..(10)
<223>  n = a, g, c or t


<400>  15
gacttggttn agtactcacc                                                    20


<210>  16
<211>  19
<212>  DNA
<213>  Artificial Sequence

<220>
<223>  probe sequence

<220>
<221>  misc_feature
<222>  (10)..(10)
<223>  n = a, g, c or t


<400>  16
atcttacggn tggcatgac                                                     19


<210>  17
<211>  22
<212>  DNA
<213>  Artificial Sequence

<220>
<223>  probe sequence

<220>
<221>  misc_feature
<222>  (12)..(12)
<223>  n = a, g, c or t


<400>  17
agaattatgc antgctgcca ta                                                 22


<210>  18
<211>  18
<212>  DNA
<213>  Artificial Sequence

<220>
<223>  probe sequence

<220>
<221>  misc_feature
<222>  (9)..(9)
<223>  n = a, g, c or t


<400>  18

gtgctgccnt aaccatga                                                    18

<210>  19
<211>  24
<212>  DNA
<213>  Artificial Sequence

<220>
<223>  probe sequence

<220>
<221>  misc_feature
<222>  (14)..(14)
<223>  n = a, g, c or t

<400>  19
tgccataacc atgngtgata acac                                            24

<210>  20
<211>  17
<212>  DNA
<213>  Artificial Sequence

<220>
<223>  probe sequence

<220>
<221>  misc_feature
<222>  (8)..(8)
<223>  n = a, g, c or t

<400>  20
cggaggancg aaggagc                                                    17

<210>  21
<211>  24
<212>  DNA
<213>  Artificial Sequence

<220>
<223>  probe sequence

<220>
<221>  misc_feature
<222>  (13)..(13)
<223>  n = a, g, c or t

<400>  21
ccgctttttt gcncaacatg gggg                                            24

<210>  22
<211>  19
<212>  DNA
<213>  Artificial Sequence

<220>

<223>  probe sequence

<220>
<221>  misc_feature
<222>  (10)..(10)
<223>  n = a, g, c or t


<400>  22
ctcgccttgn tcgttggga                                                    19


<210>  23
<211>  17
<212>  DNA
<213>  Artificial Sequence

<220>
<223>  probe sequence

<220>
<221>  misc_feature
<222>  (9)..(9)
<223>  n = a, g, c or t


<400>  23
ttgatcgtng ggaaccg                                                      17


<210>  24
<211>  19
<212>  DNA
<213>  Artificial Sequence

<220>
<223>  probe sequence

<220>
<221>  misc_feature
<222>  (10)..(10)
<223>  n = a, g, c or t


<400>  24
tgatcgttgn gaaccggag                                                    19


<210>  25
<211>  17
<212>  DNA
<213>  Artificial Sequence

<220>
<223>  probe sequence

<220>
<221>  misc_feature
<222>  (9)..(9)
<223>  n = a, g, c or t


<400>  25

caccacgang cctgtag                                                    17

<210>  26
<211>  19
<212>  DNA
<213>  Artificial Sequence

<220>
<223>  probe sequence

<220>
<221>  misc_feature
<222>  (10)..(10)
<223>  n = a, g, c or t

<400>  26
cgatgcctgn agcaatggc                                                  19

<210>  27
<211>  23
<212>  DNA
<213>  Artificial Sequence

<220>
<223>  probe sequence

<220>
<221>  misc_feature
<222>  (12)..(12)
<223>  n = a, g, c or t

<400>  27
aactattaac tngcgaacta ctt                                             23

<210>  28
<211>  22
<212>  DNA
<213>  Artificial Sequence

<220>
<223>  probe sequence

<220>
<221>  misc_feature
<222>  (12)..(12)
<223>  n = a, g, c or t

<400>  28
actattaact gncgaactac tt                                              22

<210>  29
<211>  22
<212>  DNA
<213>  Artificial Sequence

<220>

<223>  probe sequence

<220>
<221>  misc_feature
<222>  (11)..(11)
<223>  n = a, g, c or t

<400>  29
ctagcttccc ngcaacaatt aa                                                22

<210>  30
<211>  18
<212>  DNA
<213>  Artificial Sequence

<220>
<223>  probe sequence

<220>
<221>  misc_feature
<222>  (9)..(9)
<223>  n = a, g, c or t

<400>  30
agttgcagna ccacttct                                                     18

<210>  31
<211>  19
<212>  DNA
<213>  Artificial Sequence

<220>
<223>  probe sequence

<220>
<221>  misc_feature
<222>  (10)..(10)
<223>  n = a, g, c or t

<400>  31
aaatctggan ccggtgagc                                                    19

<210>  32
<211>  17
<212>  DNA
<213>  Artificial Sequence

<220>
<223>  probe sequence

<220>
<221>  misc_feature
<222>  (9)..(9)
<223>  n = a, g, c or t

<400>  32

atctggagnc ggtgagc                                                    17

<210>  33
<211>  17
<212>  DNA
<213>  Artificial Sequence

<220>
<223>  probe sequence

<220>
<221>  misc_feature
<222>  (9)..(9)
<223>  n = a, g, c or t

<400>  33
ctggagccng tgagcgt                                                    17

<210>  34
<211>  18
<212>  DNA
<213>  Artificial Sequence

<220>
<223>  probe sequence

<220>
<221>  misc_feature
<222>  (10)..(10)
<223>  n = a, g, c or t

<400>  34
ctggagccgn tgagcgtg                                                   18

<210>  35
<211>  18
<212>  DNA
<213>  Artificial Sequence

<220>
<223>  probe sequence

<220>
<221>  misc_feature
<222>  (9)..(9)
<223>  n = a, g, c or t

<400>  35
gagccggtna gcgtgggt                                                   18

<210>  36
<211>  17
<212>  DNA
<213>  Artificial Sequence

<220>

<223>  probe sequence

<220>
<221>  misc_feature
<222>  (9)..(9)
<223>  n = a, g, c or t


<400>  36
gtgggtctng cggtatc                                                    17


<210>  37
<211>  19
<212>  DNA
<213>  Artificial Sequence

<220>
<223>  probe sequence

<220>
<221>  misc_feature
<222>  (10)..(10)
<223>  n = a, g, c or t


<400>  37
gtgggtctcn cggtatcat                                                  19


<210>  38
<211>  22
<212>  DNA
<213>  Artificial Sequence

<220>
<223>  probe sequence

<220>
<221>  misc_feature
<222>  (11)..(11)
<223>  n = a, g, c or t


<400>  38
ccgtatcgta nttatctaca cg                                              22


<210>  39
<211>  18
<212>  DNA
<213>  Artificial Sequence

<220>
<223>  probe sequence

<220>
<221>  misc_feature
<222>  (10)..(10)
<223>  n = a, g, c or t


<400>  39

ttatctacan gacgggga                                                    18


<210>  40
<211>  17
<212>  DNA
<213>  Artificial Sequence

<220>
<223>  probe sequence

<220>
<221>  misc_feature
<222>  (9)..(9)
<223>  n = a, g, c or t


<400>  40
cgacggggng tcaggca                                                     17


<210>  41
<211>  21
<212>  DNA
<213>  Artificial Sequence

<220>
<223>  probe sequence

<220>
<221>  misc_feature
<222>  (11)..(11)
<223>  n = a, g, c or t


<400>  41
atggatgaac naaatagaca g                                                21


<210>  42
<211>  21
<212>  DNA
<213>  Artificial Sequence

<220>
<223>  probe sequence

<220>
<221>  misc_feature
<222>  (11)..(11)
<223>  n = a, g, c or t


<400>  42
ggatgaacga natagacaga t                                                21


<210>  43
<211>  23
<212>  DNA
<213>  Artificial Sequence

<220>

<223>  probe sequence

<220>
<221>  misc_feature
<222>  (12)..(12)
<223>  n = a, g, c or t


<400>  43
tagacagatc gntgagatag gtg                                              23


<210>  44
<211>  20
<212>  DNA
<213>  Artificial Sequence

<220>
<223>  amplification primer

<400>  44
atgagtattc aacatttccg                                                  20


<210>  45
<211>  20
<212>  DNA
<213>  Artificial Sequence

<220>
<223>  amplification primer

<400>  45
ttaatcagtg aggcacctat                                                  20


<210>  46
<211>  17
<212>  DNA
<213>  Artificial Sequence

<220>
<223>  probe sequence

<220>
<221>  misc_feature
<222>  (9)..(9)
<223>  n = a, g, c or t


<400>  46
gccttgatng ttgggaa                                                     17


<210>  47
<211>  19
<212>  DNA
<213>  Artificial Sequence

<220>
<223>  probe sequence

<220>

<221>  misc_feature
<222>  (10)..(10)
<223>  n = a, g, c or t

<400>  47
gccttgatcn ttgggaacc                                                    19

## Description

The present invention pertains to a method for a rapid detection of microbial resistance towards antibiotics and simultaneous determining the genotype of the resistance gene using a micro-array and/or to a kit for use in this method.

In the past, the continuous use of antibiotics especially in the clinical environment has increased the selective pressure for bacteria eventually leading to the development of a number of microbes exhibiting resistance towards the agents utilized. A major class of antibiotics rely on a beta-lactam-ring system against which microbes developed enzymes, the so called, beta-lactamases, which are capable of degrading the agent and have spread ubiquitously in different microbial species.

So far, over 340 different beta-lactamase sequences have been described. TEM beta-lactamases are one group of serine beta-lactamases (i.e. OXA, SHV), and are found in many different species of the family *Enterobacteiacae, Pseudomonas Aeruginosa, Haemophilus influenzae,* and *Neisseria. gonorrhoe.* The first TEM enzyme (TEM-1) was isolated in 1965 from, *E*. *coli* and confers resistance to narrow spectrum cephalosporins, cefamandole, and cefoperazone and all the anti-gram-negative-bacterium penicillins except temocillin.

In order to cope with such resistant strains, antibiotics with an extended spectrum have been developed, yet with their extensive use again resulting in the emergence of new resistances. In the meantime, some members of the TEM-, SHV- and OXA-family show the so called "Extended Spectrum Beta-Lactamase" (ESBL) phenotype which confers resistance to a variety of different cephalosporins and monolactams. Also, derivatives of the classical TEM- or SHV-enzymes appeared showing a so called "Inhibitor Resistant TEM"- (IRT) phenotype, and conferring resistance to inhibitors of beta-lactamases like clavulanic acid It has been found that the ESBL- or IRT-enzymes mainly differ in 1 to 7 positions in their amino acid sequence as compared to the parental TEM-1 sequence with 37 different amino acid substitution positions being published up to date for TEM beta-lactamases. It has also been noted that the mutations leading to an ESBL phenotype are distinct from those leading to the IRT phenotype while some mutations may occur in both phenotypes.

These resistant strains in general, and an initially unnoticed development of additional resistant strains may jeopardize the treatment and protection of a patient, especially in a clinical environment, since the attending physician may not predict, whether the antibiotic administered will prove effective in the course of the treatment. For this reason, the knowledge about the presence of resistant bacteria is of utmost importance for the decision, which antibiotic is to be used.

The clinical standard procedures for identifying pathogens and a potential resistance are tedious and may require up to three days before a resistance can be unequivocally determined. These procedures mostly rely on phenotypic identification using agar diffusion tests, which are cost-effective though, but slow (up to 48 hours). Also, multiple antibiotics have to be tested in order to identify an ESBL phenotype.

However, such a long time-lag between sampling and obtaining the results of the experiments on the basis of which the appropriate antibiotic may be selected does put the patient in danger and may, in severe cases, even put his life on stake. For this reason, under normal clinical circumstances, the patient receives an "up-front'' administration of a broad-spectrum antibiotic, which is prospected to exert at least some efficacy. Proceeding accordingly will, however, even contribute to the development of more resistant strains.

Thus, there is clearly a need in the art for a method which permits both, a rapid and highly reliable determination of a potential resistance present in a micro-organisms contained in a biological sample, and also an easy processing of a plurality of samples. In addition, due to the high cost pressure in the medical field, performing the method should not be cost-intensive.

This problem has been solved by providing a method for detecting the presence or absence of a micro-organism in a biological sample exhibiting resistance to a beta-lactam antibiotic and simultaneously determining the genotype of the beta-lactam resistance, which method comprises the following steps: (i) obtaining a biological sample; (ii) optionally isolating DNA contained in the sample; (iii) contacting the DNA of the sample with a micro-array, harboring on predetermined locations thereon different sets of capture probes, under conditions allowing hybridization of complementary strands. Each representative of a set of capture probes comprises the sequence R¹-(X)-R², which sequence represents a selected part of the sequence of a beta-lactamase gene, wherein X represents a nucleotide triplet and its permutations, and wherein R¹ and R² each have a length of from about 5 to 20 nucleotides, wherein the different sets of capture probes are selected such that an adjacent set starts at a given position 3n of nucleotides down-stream from the first set of capture probes, wherein n is an integer of 1 to 10, so that the nucleotide sequence of the beta-lactamase gene is covered over a desired range. The method furthermore comprises the step (iv) determining whether and hybridization occurs and at which position on the array, wherein the occurrence and location of a hybridization is indicative of the presence of a beta-lactam resistant micro-organism and also indicative of its specific resistance.

In the figures,

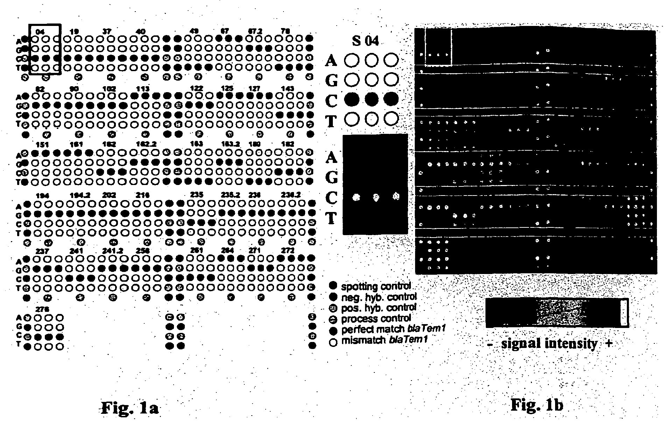

Figure 1a shows a SNP capture probe layout of a TEM micro-array. All SNP capture probes are spotted in triplicates. The SNP position is named after the position in the encoded amino acid sequence and is indicated above each triplicate. SNP capture probe set 04 is highlighted and shown enlarged on the right side. The varying, essentially centrally located base in the probe sequence is indicated on the left side. Blue spots indicate perfect match positions for *blaTEM-1* beta-lactamase gene. Controls included on the array: a probe labeled with Cy5 (cyanine5) at the 5' end as a spotting control, a positive hybridization control and a negative hybridization control. All three control sequences are unrelated to bacterial species. The process control consists of a conserved sequence within the *blaTEM* gene family.

Figure 1b is a fluorescence image of a hybridization experiment with 400 ng of *blaTEM-1* target DNA (number of nucleotides%incorporated fluorescent dye = NT/F = 83) for 3 hours in a hybridization station. All the perfect match positions for *blaTEM-1* can be identified by the highest signal intensity within one capture probe set. SNP capture - probe set S04 is highlighted and shown enlarged. The signal intensity is shown in false color, blue corresponds to the lowest signal intensity, red to white depict the highest signal intensities.

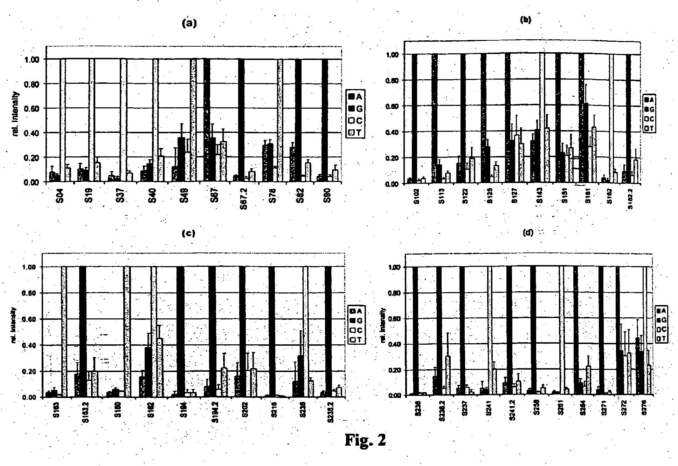

Figure 2 shows the result of hybridization with *blaTEM-1* (n=5) (in each hybridization 400ng of target DNA were applied, the labeling efficiencies varied: NT/F = 71-180) for 3 hours in the hybridization station. Shown here is the relative intensity, which corresponds to the ratio of mismatch or perfect match signal intensity/perfect match signal intensity [(MM or PM)/PM], so the relative (rel.) intensity of the perfect match corresponds to 1. The mismatch relative intensities are < 1. All the perfect match positions for *blaTEM-1* could be identified correctly (n = number of hybridization experiments).

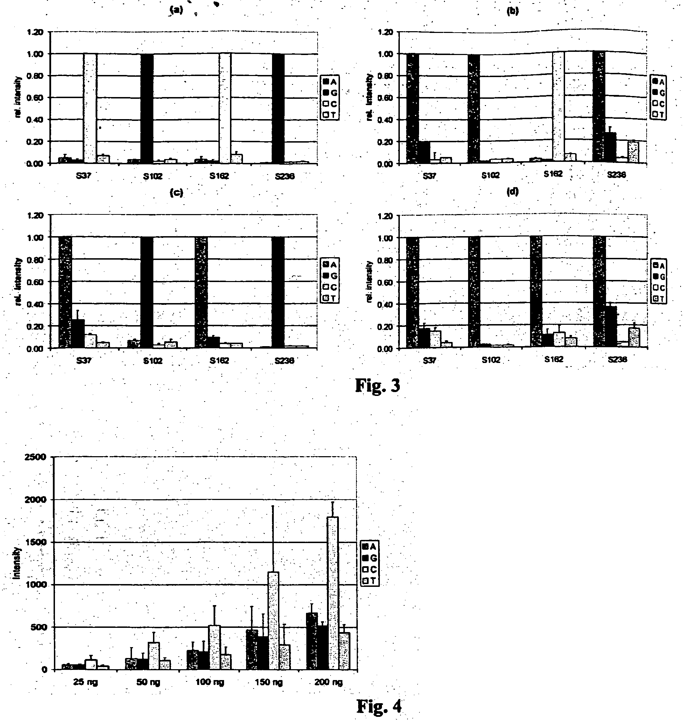

Figures 2a, 3b, 2c, and -2d show the relative signal intensities of SNP positions S04-S90, S102-S162.2, S163-S235.2, and S236-S276; respectively.

Figure 3 shows an identification of perfect match positions of SNP positions 37, 102, 162, 236 of (a) *blaTEM-1* (n = 5) and (b) *blaTEM-3* (n - 2) (c) *blaTEM-7* (n *=* 2) *and* (d) *blaTEM-8* (n = 3). The relative intensity is given as the ratio of signal intensity/perfect match signal intensity [(MM or PM)/PM]. The mutated amino acid positions in comparison to *blaTEM-1* are: S37 (CAG to AAG, Q to K) in *blaTEM-3*,*-7*,*-8*; S102 (GAG to AAG, E to K) *in blaTEM-3*,*-8*; S162 (CGT to ACT, R to S) in *blaTEM-7,-8,* S236 (GGT to AGT, G to S) in *blaTEM-3,* -*8*. The other perfect match positions are identical to *blaTEM-1* perfect match positions (data not shown). (n = number of hybridization experiments)

Figure 4 shows the determination of the detection limit on the array. Shown here is the result of hybridization of 25-200ng of DNA of blaTEM-116 with a cover glass in Coming hybridization chambers (n =3). The labeling efficiency (NT/F) of the hybridized sample DNA was 87. The displayed signal intensities are those of SNP position S235, which showed the weakest perfect match signal intensity after hybridization from all detected SNP positions. The perfect match position for *blaTEM-116* at S235 is C, mismatch positions are A, G and T. For 25 ng DNA the perfect match position can still be discriminated from the mismatch position, but the signal intensity is very weak (< 200): (n = number of hybridization experiments)

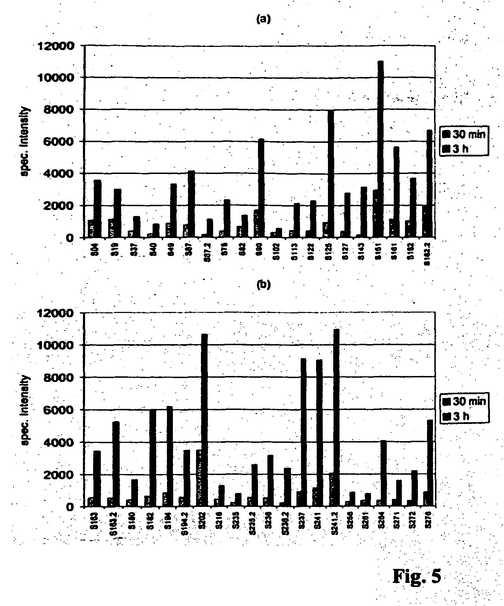

Figure 5 shows a result of the hybridization of 65 ng DNA (NT/F = 57) at different incubation times: 30 min and 3 hours. Shown here are the specific intensities, which correspond to the difference between perfect match intensity and the mean of the mismatch intensities [PM-(MM₁+MM₂+MM₃)/3].

Figures 5a, 5b show specific intensities of SNP positions S04-S162.2, and S163-S276, respectively. The specific intensities at 30 min incubation time are up to 10 x weaker, but still all perfect match positions can be identified.

### In the present description the following definitions apply:

The terms "micro-array" and "array of oligonucleotides'' may be used interchangeably in the present invention and refer to a multiplicity of different nucleotide sequences attached (preferably through a single terminal covalent bond) to one or more solid supports where, when there is a multiplicity of supports, each support bears a multiplicity of nucleotide sequences. Both terms can refer to the entire collection of oligonucleotides on the support(s) or to a subset thereof. The support is generally composed of a solid surface which may be selected from the group consisting of glasses, electronic devices, silicon supports, silica, metal or mixtures thereof prepared in format selected from the group of slides, discs, gel layers and/or beads.

As used in present invention, the term "probe" is defined as an oligonucleotide capable of binding to a target nucleic acid of complementary sequence through one or more types of chemical bonds, usually through complementary base pairing, usually through hydrogen bond formation. As used herein, an oligonucleotide probe may include natural (i.e. A, G, C, or T) or modified bases (7-deazaguariosine, inosine, etc.). In addition, the bases in oligonucleotide probe may be joined by a linkage other than a phosphodiester bond, such as e.g. peptide bonds, so long as it does not interfere with hybridization.

The term "target nucleic acid" refers to a nucleic acid, to which the oligonucleotide probe specifically hybridizes.

The term "capture probes" in the sense of the present invention shall designate parts of the beta-lactamase gene of different length, e.g. between about 10 and 43 nucleotides, which are either chemically synthesized in situ on the surface of the support or laid down thereon.

The term "perfect match probe" refers to a probe that has a sequence that is perfectly complementary to a particular target sequence.

The term "beta-lactamase" as used in the present application comprises all currently known classes of beta-lactamases, such as e.g. serin- or a zink- beta-lactamases, and e.g. comprises in particular TEM beta-lactamases which form a subgroup of plasmid mediated serine beta-lactamases (such as e.g: OXA, SHV).

The term "nucleotide sequence" as used herein refers to nucleotides present in nucleic acids compared with the bases of said nucleic acid, and includes nucleotides comprising usual or modified bases as above described. References to nucleotide(s), oligonucleotide(s), polynucleotide(s) and the like include analogous species wherein the sugar-phosphate backbone is modified and/or replaced, provided that its hybridization properties are not destroyed.

The phrase "hybridizing specifically to" refers to the binding, duplexing or hybridizing of a molecule substantially to or only to a particular nucleotide sequence or sequences under stringent conditions when that sequence is present in a complex mixture of DNA or fragments thereof.

The term "essentially simultaneous detection on a micro-array" as used in the present invention indicates that one or more target sequences may be screened for on a micro-array, the presence or absence thereof being determined essentially simultaneously depending on the specifically applied preparation and reading methods, etc..

During the extensive studies leading to the present invention the inventors devised a new way to determine the presence or absence of a micro-organism harboring a beta-lactamase exhibiting resistance to particular beta-lactam antibiotics.

To this end, the micro-array put to use in the present method contains on predetermined locations thereon a plurality of sets of nucleotide sequences of the general formula R₁-(X)-R₂. A set of nucleotide sequence contains three main components, R₁, X and R₂. R₁ and R₂ are nucleotide sequences having a length of from about 3 to about 25 nucleotides and are derived from a particular sequence of interest of a beta-lactamase gene of a micro-organism. These sequences R₁ and R₂ are separated by a triplet in the original beta lactamase gene, which is designated with "X". In order to screen for variations at a or the specific location (i.e. "X") the triplet is varied and may cover all 64 permutations for the amino acids. However, for practical purposes the variation of "X" is selected such, to account for a potential change in an amino acid in the gene sequence For example, in case the original triplet was GCT, encoding the amino acid alanine, the triplets GCC, GCG and GCA may in principle be disregarded, since such mutations would not result in a change of the primary structure of the resulting polypeptide, which eventually will account for the development of a resistance towards a particular drug. Therefore, a set of nucleotides covers a specific position of interest in the beta-lactamase gene.

On the micro-array a plurality of different such sets are arranged so as to cover a plurality of different locations of interest in the beta-lactamase gene. In principle and according to a broad view, the micro-array provides a number of sets of nucleotides, wherein adjacent to a given set of nucleotide - sequence R₁-X-R₂ (wherein e.g. R₁ is ATG encoding the start methionine, X is the second triplet in a beta-lactamase gene, and R₂ is a nucleotide sequence downstream of the said 2^{nd} triplet), the next nucleotide sequence R₁-X-R₂ is shifted downstream by three nucleotides (e.g. R, is ATG plus the next three original nucleotides, X is the triplet spaced by three nucleotides from the start codon ATG, and R₂ is a nucleotide sequence shifted by three nucleotides downstream). To this end, such an micro-array allows a "gene-walking" and a determination of any mutations in the beta-lactamase, gene.

Such a micro-array allows the determination, whether or not a micro-organism, that contains a beta-lactamase gene, is present in the biological sample. In addition, since the position of the mutation in the beta-lactamase gene has been determined simultaneously, it is also possible to determine, in case the mutation is known and has been linked with a specific resistance already, to which antibiotic the micro-organism is resistant. The attending physician may, therefore, immediately take the necessary steps and administer an agent, known to be effective.

The present invention also provides information about mutations having occurred in the beta-lactamase gene not yet known. In this case, the attending physician does not know prima facie, whether ornot the mutation has led to the development of a new resistance towards a particular agent. However, based on the information provided in the present method, he may now carry out conventional experiments testing the sensitivity of the micro-organism. Therefore, the present invention provides the opportunity to essentially prevent the spread of micro-organisms, having developed new resistances, since the resistance may be detected/determined at an early stage and treated accordingly.

When performing the method of the present invention, a biological material is used as a starting material that may contain one or more (different) micro-organisms deemed to exhibit resistances. Examples of appropriate samples are blood samples, tissue samples, body secrets, such as sputum, etc. or culture media samples comprising microorganisms. For certain applications, it may be appropriate to transfer the biological sample to a medium suitable for the growth of micro-organisms, e.g. on plates (for example Müller-Hinton agar plates).

The biological sample may be utilized as such or DNA may be isolated therefrom according to a method of the state of the art, such as an extraction according to the QIAprep® Spin Miniprep Kit protocol (Qiagen, Hilden, Germany). Alternative appropriate methods for obtaining DNA may be chosen, depending on the specific starting material on the basis of the knowledge of a skilled person in the art. Such an isolation step assists in preventing the development of extensive background signals during the hybridization step, in case no other selection step is applied.

According to an embodiment the DNA contained in the biological sample or isolated may be amplified via a polymerase chain reaction (PCR) using one or more primers, which provides the advantage of augmenting the specific material to be investigated only and also to incorporate a selection step. In case of using one primer only, the complementary strand of the DNA of interest will be synthesized. Alternatively, at least two primers are utilized, allowing an exponential amplification of the material to be investigated. According to an alternative embodiment a nested PCR is carried out, wherein 2 pair of primers are used. The first set of primers is designed to amplify a sequence around the target sequence. Then the second pair of primers are used to amplify a sequence lying within the amplified sequence. After the completion of the PCR reaction, the PCR product may be purified if desired.

When using an amplification step; the DNA may at the same time be labeled, e.g. by including in the amplification process nucleotides harboring an appropriate label. Alternatively, a label may be attached to the nucleic acid, including, for example, nick translation or end-labeling by attachment of a nucleic acid linker joining the sample nucleic acid to a label.

Detectable labels suitable for use in the present invention include any composition detectable by spectroscopic, photochemical, biochemical, immunochemical, electrical, optical or chemical means. Useful labels in the present invention include biotin for staining with labeled streptavidin conjugate, magnetic beads (e.g., Dynabeads^{TM}), fluorescent dyes (e.g., cyanine dyes, such as Cy5, fluorescein, texas red, rhodamine, green fluorescent protein, and the like), radiolabels (e.g., ³H, ¹²⁵I, ³⁵S, ¹⁴C, or ³²P), enzymes (e.g., horse radish peroxidase, alkaline phosphatase and others commonly used in an ELISA), and colorimetric labels such as colloidal gold or colored glass or plastic (e.g., polystyrene, polypropylene, latex, etc.) beads. Patents teaching the use of such labels include U.S. Patent Nos. 3,817,837; 3,850,752; 3,939,350; 3,996,345; 4,277,437; 4,275,149; and 4,366,241.

In order to allow detection of the presence of a single mutation, the target- or probe-DNA should not be too long, since otherwise renaturation in solution, or base-pairing with a capture probe allowing mismatches may occur. The desired length of such a probe DNA should be of from about 10 to 50 nucleotides, preferably 15 to 40, more preferably 15 -30, even more preferred 15- 25 nucleotides and may be obtained by either selecting the primers during the amplification step accordingly, or by fragmenting the DNA put to use after an amplification step.

The target-/probe-DNA thus obtained is the contacted with the capture probes on the micro-array under conditions allowing hybridization of complementary strands only.

The capture probe, associated with the micro-arrays, may be derived from any of the beta-lactamase genes known so far, such as e.g. from the beta-lactamase gene of Enterobacteiacae, e.g. *E.coli, Enterobacter spp., Morganella morganii, Proteus* spp., *Providencia* spp. *Salmonella* spp., *Klebsiella spp., Citrobacter spp., Shigella dysenteriae, Serratia marcescens,* and non fermenting bacteria, i.e. *Pseudomonas spp., Burkholderia cepacia, and other gram negative species of pathogenic relevance, e.g. Haemophilus spp., Neisseria spp.* (Bradford, Clinical Microbiology Reviews; Oct.2001, p933-951; Thomson, Emerging Infectious Diseases, March-April 2001, p333-336). The references for the published sequences of the different genes are in public domain may be derived from http://www.lahey.org/, which documents are incorporated herein by way of reference.

Alternatively, in order to provide a specific view to known resistances, the capture probes are selected, so that the triplet to be permutated covers known locations of mutations in a beta-lactamase gene, for example TEM beta-lactamase, SHV beta-lactamase, OXA beta-lactamase, K1 beta-lactamase in particulat mutations correlated with an Extended Spectrum (ESBL) phenotype or an Inhibitor Resistant TEM (IRT) phenotype.

According to another preferred embodiment the capture probes on the array are selected such that they cover known locations of SNP's (Single Nucleotide Polymorphisms) in beta-lactamase genes. Table I lists a number of polymorphism sites and the corresponding TEM mutants.

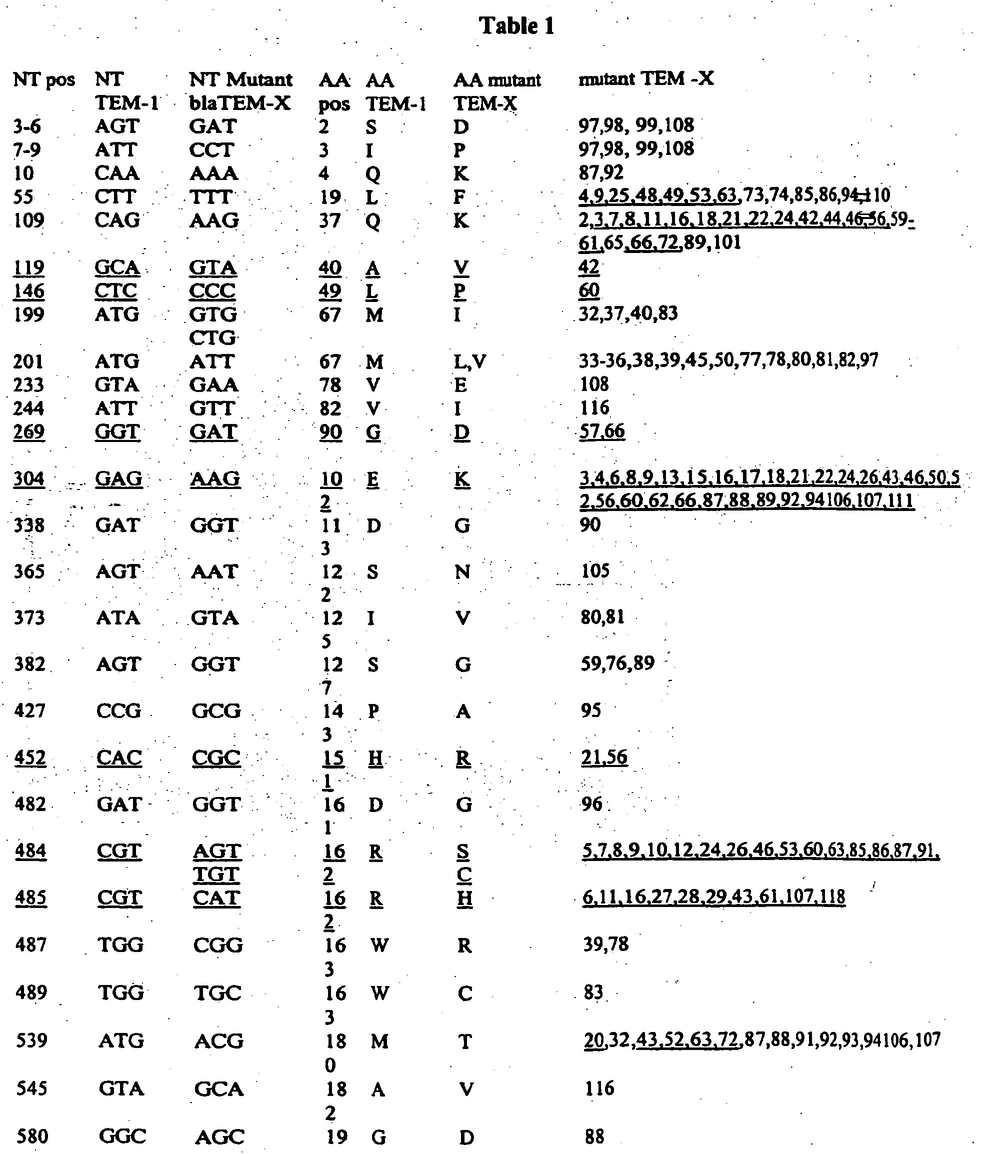

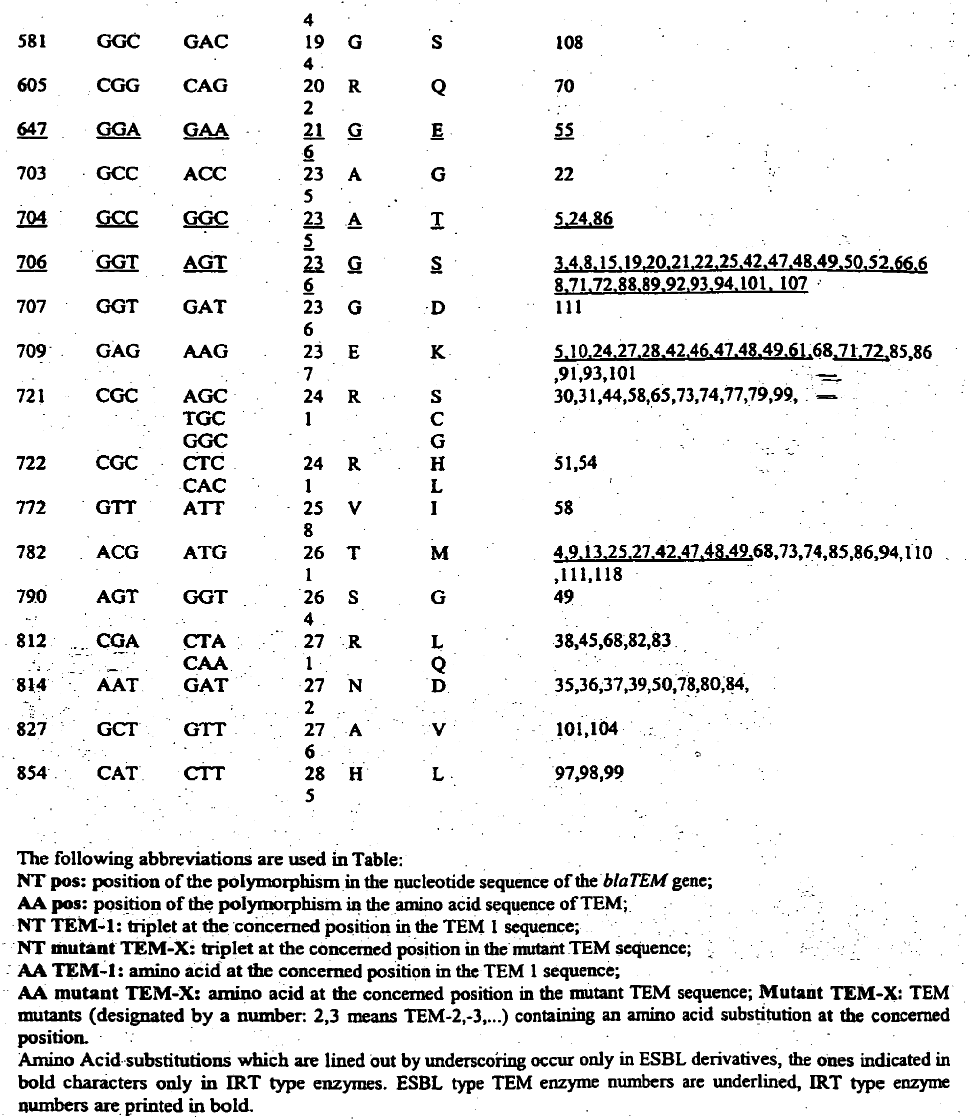

As may be derived from Table 1 above, some mutations occur in both phenotypes. In contrast to the phenotypic identification methods for beta-lactamases of the state of the art, the method according to the present invention on the basis of genotyping processes permits to monitor the spread of different resistance genes throughout the clinical setting, and may thereby provide valuable information for the development of strategies preventing this propagation.

Hybridization processes and conditions therefore are well known in the art and are described e.g. in Current protocols in Molecular Biology (2000); Chapter 22, John Wiley & Sons, Inc..

E.g. the hybridization step may be performed under a cover glass in hybridization chambers or in a hybridization oven which essentially represent a diffusion limited process. Alternatively, the reaction may be carried out with agitation, which decrease the required hybridization time. When using hybridization involving movement of the micro-array, the hybridization time may be reduced to a period of 60 minutes or less, preferably to 30 minutes or less. As a result thereof, the total assay can be performed in less than 4, more preferably 3.5 hours. This is in particular of high interest, as it permits to obtain the results on the same day a patient arrives at a hospital or contacts a physician.

Subsequently, the presence or absence of one or more specific hybridizations will be determined, which will eventually indicate the presence or absence of one or more beta-lactamase encoding nucleotide sequences and thus the presence or absence of a beta-lactam resistant micro-organism.

This may be achieved by detecting the label attached to the target-DNA prior to the hybridization step, or by performing the labeling reaction on the array. So called "direct labels" are detectable labels that are directly attached to or incorporated into the target (sample)nucleic acid prior to hybridization. In contrast, so called "indirect labels" are joined to the hybrid duplex after hybridization. The indirect label may also be attached to a binding moiety that has been attached to the target nucleic acid prior to the hybridization. For a detailed review of methods of labeling nucleic acids and detecting labeled hybridized nucleic acids see Laboratory Techniques in Biochemistry and Molecular Biology, Vol. 24: Hybridization With Nucleic Acid Probes, P. Tijssen, ed. Elsevier, N.Y., (1993)).

Also, the capture probes present on the array may contain a label at their 3'-end. After binding of the target DNA, the DNA/DNA hybrids are then cleaved with a particular enzyme thus releasing the label from those capture probes, where the target DNA had bound. Therefore, in this embodiment, the decrease in signal is representative of the presence of a beta-lactamase gene in the sample.

In principle, means of detecting labeled target nucleic acids hybridized to probes are known to those of skilled in the art. For example, where a colorimetric label is used, simple visualization of the label is sufficient. Where a radioactive labeled probe is used, detection of the radiation, e.g. with photographic film or a solid state detector is sufficient. In a preferred embodiment, however, the target nucleic acids are labeled with a fluorescent label and the localization of the label on the probe array may be accomplished e.g. with fluorescent microscopy. The hybridized array is excited with a light source at the excitation wavelength of the particular fluorescent label and the resulting fluorescence at the emission wavelength is detected. In a particularly preferred embodiment, the excitation light source is a laser appropriate for the excitation of the fluorescent label.

The imaging system (confocal microscope) may be automated with a computer-controlled stage to automatically scan the entire high density array. Similarly, the microscope may be equipped with a phototransducer (e.g., a photomultiplier, a solid state array, a CCD camera, etc.) attached to an automated data acquisition system to automatically record the fluorescence signal produced by hybridization to each oligonucleotide probe on the array.

In a most preferred embodiment, the present method puts to use an oligonucleotide micro-array for identifying single nucleotide polymorphisms (SNP's) of 119 until today described TEM beta-lactamases (http://www.lahey.org/Studies). This micro-array contains for 41 SNP (single nucleotide polymorphism) positions (ESBL, IRT, or both) oligonucleotide probes with variable length (17-27 bases) (cf. table 2).

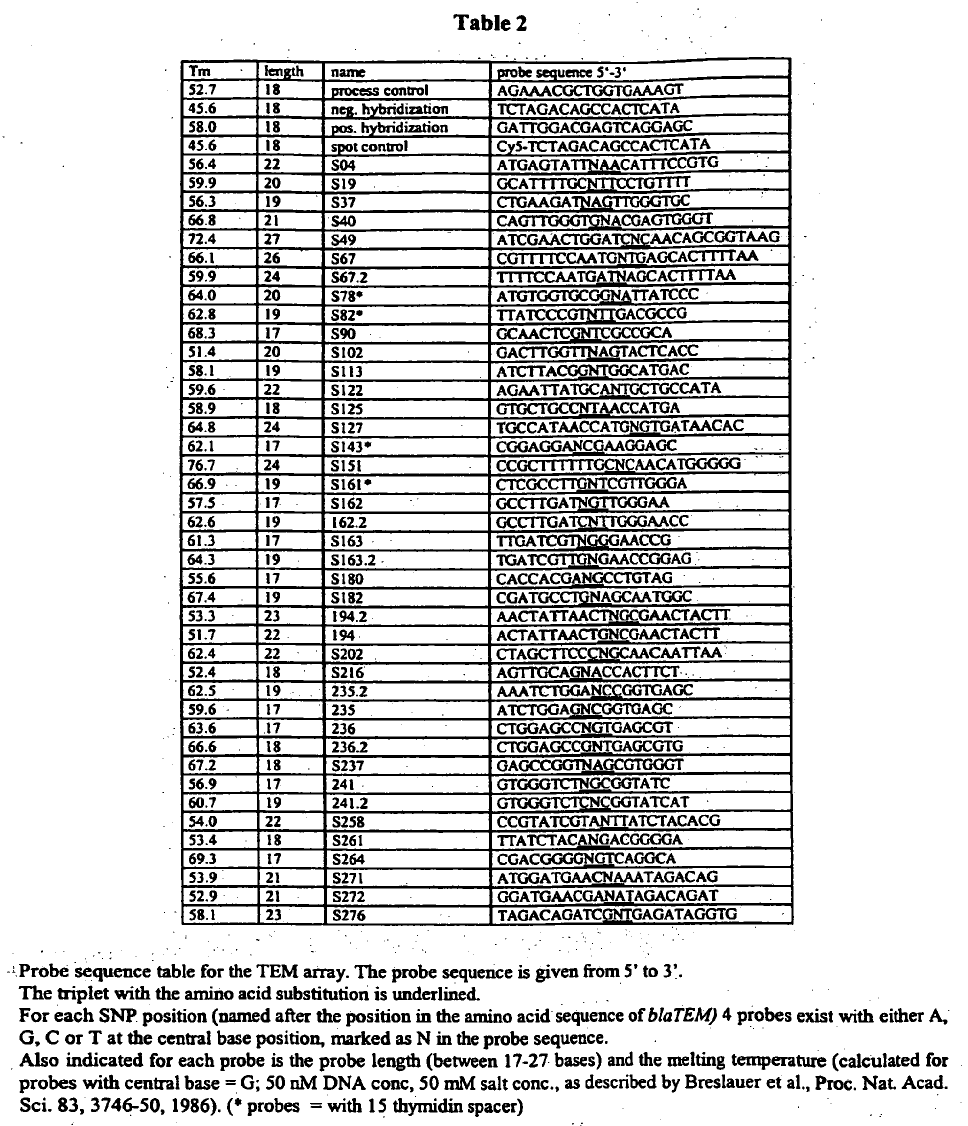

These probes cover most of the amino acid substitution positions published up to date (www.lahey.org/studies/web.htm) except the end-standing positions of amino acids number 2, 3 and 285 of TEM beta-lactamase. The probes were designed with the SNP at the central base within the probe sequence for maximum perfect match/mismatch discrimination during hybridization. For each SNP a probe set of 4 probes is designed with identical sequence except the central base, which is either A,T, G or C. The SNP probes are named after the position of the amino acid substitution within the TEM sequence, that means S161 means the polymorphism at position 161 in the TEM amino acid sequence. For some amino acid substitution positions there are two polymorphisms within one triplet, for these two SNP probe sets (2 x 4 probes) are designed (e. g. S162, 162.2). Several controls are also included on the array: a spotting control, which is a probe with a Cy5 label at the 5' end, a positive hybridization control complementary to a labeled oligonucleotide target, which is spiked in during hybridization and a negative hybridization control. All these controls consist of sequences unrelated to bacterial species. The process control corresponds to a conserved sequence within the *blaTEM* gene family. Each SNP probe may be spotted in triplicate. The controls may be spotted multiple times and distributed over the array surface. The array layout is shown in figure 1a, a fluorescence image of a TEM array hybridized with *blaTEM-1* target DNA in 1b.

For using the micro-array in the present method, the isolated plasmid DNA from clinical samples is amplified and labeled during PCR with primers flanking the *blaTem* gene, yielding a 861 bp fragment.

The incorporation ratio of the label (number of nucleotides incorporated) is determined. The target DNA is fragmented with DNase1 to fragment sizes of about 15 to 150 bp in order to increase the efficiency of hybridization. The array is then hybridized with the target DNA and the hybridization signals are acquired with an appropriate device, e.g. a scanner (figure 1b).

The overall assay time with 2 hours for amplification, 30 min for purification and labeling, 3 hours for hybridization and 30 minutes for wash, is 6 hours. Since for the application of this array in the clinical setting the assay should be as fast as possible, the present method allows a hybridization time to as low as 30 min. The corresponding data is shown in figure 5. Even though the specific intensities at 30 minutes incubation time are weaker as expected, all perfect match positions could still be identified unequivocally.

The hybridization step may be carried out under a cover glass, so the hybridization in this case is a diffusion limited process. Also, hybridization may also be performed under agitation of the hybridization solution which yields higher intensities, so that the hybridization can be reduced to less than 30 minutes even providing acceptable intensities. To this end the total assay time may be reduced to less than 3,5 hours.

The present method allows for a reproducible and sensitive identification of various genes of ESBL type beta lactamase. With an assay time of less than 3 to 4 hours, this method is a very fast detection method for ESBL and/or IRT TEM beta-lactamase and is a valuable tool to monitor the spread of these resistance genes e.g. within the clinical setting.

In order to provide a desired reliability, the micro-array comprises at least one of a spotting control, a positive hybridization control, a negative hybridization control, and/or a process control.

An additional advantage of the present invention is that it permits even to detect two polymorphisms present in one triplet, as in this case two sets of SNP probes may be designed and will be present on the micro-array.

The present invention also provides a kit for detecting the presence or absence of a plurality of beta-lactamase encoding nucleotide sequences and/or for determining the presence or absence of a plurality of beta-lactam resistances / mutations in the nucleotide sequences in one or more microorganisms, said kit comprising: a micro-array as described above and optionally buffers and reagents suitable for carrying out the present method.

Both the method and the kit according to the present invention may be used for designing a pharmaceutical composition for treating a patient in need of a treatment with antibiotics. In particular a pharmaceutical composition could be designed comprising a specific antibiotic, in particular one or more beta-lactam(s) and if necessary, one or more additional beta-lactamase inhibitor(s) or choosing an antibiotic of different mode of action.

The present invention thus permits to provide to a patient a specifically tailored antibiotic treatment with a minimum delay, for example in less than one working day, e.g. in particular in about 3.5 hours. As a result a more effective treatment (on basis of the provision of a more effective pharmaceutical composition) may be offered to a patient. As a result thereof, it may be expected that also the duration and/or severity of the disease may be largely decreased.

The method and the kit of the present invention may be used in automated or partially automated devices, so that the working costs may be kept essentially low.The present invention provides thus also a method and a kit permitting a rapid microbial antibiotic resistance detection which may be performed at large scale (if desired) and at essentially low costs.

Additional features and advantages of the present invention are described in, and will be apparent from, the following detailed description of the invention and the figures.

By way of example, and not limitation, examples of the present invention will now be given.

### Example 1 :

### Design of a micro-array

### 1. Oligonucleotide array fabrication

Oligonucleotide arrays were constructed with 168 oligonucleotide capture probes with varying length from 17-27 bases. The capture and control probe sequences are given in Table 2 (supra) , probes equipped with a 15 thymidine spacer are marked with a star. The array layout is shown in Figure 1.

The oligonucleotide micro-array was developed for genotyping TEM beta lactamases. For 41 SNP (single nucleotide polymorphism) positions (ESBL, IRT, or both) oligonucleotide probes were designed with variable length (17-27 bases) (cf. Table 2). These probes cover most of the amino acid substitution positions currently known in the art except the end-standing positions of amino acids number 2, 3 and 285 ofTEM beta-lactamase.

The probes were designed with the SNP at the essentially centrally located base within the probe sequence for maximum perfect match/mismatch discrimination during hybridization. For each SNP a probe set of 4 probes was designed with identical sequence except the essentially centrally located base, which is either A,T, G or C. The SNP probes are named after the position of the amino acid substitution within the TEM sequence, that means S161 indicates the polymorphism at position 161 in the TEM amino acid sequence. For some amino acid substitution positions there are two polymorphisms within one triplet, for these two SNP probe sets (2 x 4 probes) were designed (e. g. S162, 162.2). Several controls are also included on the array: a spotting control, which is a probe with a Cy5 label at the 5' end, a positive hybridization control complementary to a labeled oligonucleotide target, which is spiked in during hybridization and a negative hybridization control. All these controls consist of sequences unrelated to bacterial species. The process control corresponds to a conserved sequence within the *blaTEM* gene family. Each SNP probe is spotted in triplicate. The controls are spotted multiple times and are distributed over the array surface. The array layout is shown in Fig. 1a, a fluorescence image of a TEM array hybridized with *blaTEM-1* target DNA in Fig. 1b.

The probes were dissolved in S1 spotting buffer (Eppendorf AG, Hamburg, Germany) at 20 µM concentration and spotted. with the Microgrid II (BioRobotics, UK) on CreativChip® Oligo-Slides (Eppendotf AG, Hamburg, Germany): The spots size was about 150 µm and the spot to spot distance 230 µm. For permanent immobilization the oligonucleotide array was incubated at 120°C. The blocking followed according to the CreativChip® protocol.

### 2. DNA isolation

The bacterial strains were inoculated on Müller-Hinton agar plates at 37°C over night. One loop of bacterial cells was dissolved in 250 µl buffer P1 and the plasmid DNA was extracted according to the QIAprep® Spin Miniprep Kit protocol (Qiagen, Hilden, Germany).

### 3. Amplification, labeting and purification of blaTEM

Target DNA was synthesized by PCR. The amplification primers for the blaTEM genes were temforw (5'-atgagtattcaacatttccg-3') and temrew (5'-ttaatcagtgaggcacctat-3'). For PCR amplification and labeling 1-75 ng of plasmid DNA was supplemented with 100 µl PCR-buffer (2.5 mM Mg(OAc)₂, SO mM KCl, 10 mM Tris-HCl pH 8.3) containing 50 µM dATP, dGTP, dTTP, 30 µM dCTP, 20 µM Cy5-dCTP; Amersham Biosciences, Little Chalfont, UK) and 10 U Taq DNA Polymerase (Eppendorf AG, Hamburg, Germany). Amplification was performed in a Mastercycler Gradient (Eppendorf AG, Hamburg, Germany). An initial denaturation step (94°C for 1 min) was followed by 30 cycles (94°C for 30 s, 55°C for 30 s, 72°C for 1 min) and a final extension step at 72°C for 4 min.

The PCR product was purified with the Qiaquick® Spin PCR Purification Kit (Qiagen, Hilden, Germany) according to the manufactor's protocol. The DNA. was eluted in 30 µl double distilled H₂O.

### 4. Fragmentation

The amplified and labeled target DNA was fragmented in a concentration of 30 ng/µl with 0.0115 U/µl of Dnase I (Promega, Madison, USA) at room temperature for 5 min in reaction buffer (40mM Tris-HCl, pH 8, 10 mM MgSO₄, 1 mM CaCl₂): The reaction was stopped by the addition of 3 mM EGTA and incubation at 65°C for 10 min.

As a result, the target DNA is fragmented by the DNase1 to fragment sizes of about 15 to 150 bp, whereby the efficiency of hybridization is increased.

### 5. Hybridization

Two hybridization approaches have been realized:

### Hybridization variant I

Various amounts of fragmented target DNA (25-400 ng) with addition of control DNA (0.05 pmol) were hybridized under a 18x18 mm cover glass in 30 µl of 6 x SSPE, incubated in hybridization chambers (Coming, NY, USA) and in a hybridization oven (OV5, Biometra, Gottingen, Germany) for 3 hours at 45°C.

After hybridization the slides were washed with 2 x SSC, 0.1% SDS, then 2 x SSC and finally 0.2 x SSC each time for 10 minutes at room temperature with agitation and dried with N₂.

### Hybridization variant II

Hybridization was also carried out with the hybridization station HS 4800- (Tecan, Crailsheim, Germany) with an initial wash step at 45°C with 6 x SSPE for 30 s, followed by the probe injection (400ng target DNA, 0.05 pmol control DNA in 65 µl 6 x SSPE) and hybridization under medium agitation intensity for 3 hours at. 45°C. The slides were then washed at room temperature for 2 times 2 min in 2 x SSC 0.1 % SDS and for 1.5 min in 0.2 x SSC. The slides were dried with N₂ for 2 min.

Figure 1b shows as an example an array after hybridization with target DNA, the hybridization signals being acquired with a fluorescence scanner. The array was hybridized with 5 different *blaTEM-1* samples with varying incorporation ratios (NT/F = 76-180) with the same amount of target DNA (400 ng) for 3 hours in a hybridization station.

### 6. Array analysis

Hybridization signals. were acquired with a GMS 418 Array Scanner (Affymetrix, Santa Clara, USA) and the signal intensities were quantified by Imagene™, Version 3.0 (Biodiscovery Inc:, Los Angeles, USA).

The signal intensities were acquired and the subtraction of the local background yielded the net signal intensities. These were used for calculating the mean net intensity of the triplicates on one array. The mean net intensities of the same probes on different arrays hybridized with different target DNA samples could vary with a factor bigger than three, due to the differences in the incorporation ratio or efficiency of fragmentation. Since it should be determined, if the ratio of mismatch to perfect match signal intensity remains reproducible, the relative intensity was calculated. This corresponds to the ratio of mismatch or perfect match signal intensity/perfect match intensity [(MM or PM)/PM] within one SNP probe set. Accordingly all the perfect match rel. intensities correspond to 1, the mismatch relative intensities should be < 1. The mean and the standard deviation of the relative intensities (n = 5) are given in Fig. 2. All the perfect match positions for *blaTEM-1* could be identified correctly in every sample. The mean mismatch relative intensities vary, most of them remain below 0.4, only S143, S161, S182, S276 show higher values up to 0.6. The standard deviation varies from 0.01 to 0.21. For most positions the [(MM or PM)/PM] ratio is reproducible < +/- 10%. Only for Positions 127, 235, 236.2, 276 the variation is slightly higher, but the perfect match identification is without ambiguity. The incorporation ratio of the fluorophore (number of nucleotideslincorporated fluorescent dye) is determined by OD measurement.

The array was further validated with different *blaTEM* gene variants as target DNA, e.g. *blaTEM-1,-3,-7,-8,-116.* The results of the identification of *blaTEM-1,-3,-7,-8,* are given in Figure 3 as the relative intensity values. Only the SNP positions, which varied in comparison to *bla-TEM-1* are displayed. The mutated amino acid positions in comparison to *blaTEM-1* are: S37 (CAG to AAG, Q to K) in *blaTEM -3,-7,-8;* S102 (GAG to AAG, E to K) in *blaTEM* -*3,*-*8;* S162. (CGT to AGT, R to S) in *blaTEM* -*7,* -*8*, S236 (GGT to AGT, G to S) in *blaTEM* -*3,* -*8.* The discriminative power of the relevant SNP position is distinct; since the perfect match/mismatch ratio is always > 2.5.

### 7. Detection limit

The next step was to determine the detection limit of the target DNA on the array. 25 to 200 ng of the same amplified and fragmented *blaTEM-116* target DNA (NT/F = 87) were hybridized for 3 hours in Coming hybridization chambers. As expected the signal intensities increase with a higher amount of target DNA. The displayed net signal intensities in Figure 4 correspond to SNP 235, which showed the weakest signal intensity after hybridization from all detected SNP positions in this experiment. The perfect match position for *blaTEM*-*116* at position S235 is C, mismatch positions are A, G and T. For 25 ng of target DNA the perfect match position can still be discriminated from the mismatch position. The ratio of perfect match/mismatch is still > 2, but the net signal intensity is quite low (< 200). Since the discrimination of the probe signal from the background signal is more difficult with low signal intensities the perfect match identification becomes more unreliable. The detection limit is between 25-50ng target DNA (NT/F =87).

### 8. Assay time using hybridization variant III

Hybridization variant III permits to reduce the hybridization time from 3 hours to 30 min. The array was hybridized with 65 ng of *blaTEM-116* target DNA (NT/F =57) for 30 minutes and for 3 hours in Coming hybridization chambers. The corresponding data is shown in Figure 5. The results are shown as specific intensities, which corresponds to the difference between the net perfect match intensity and the mean of the net mismatch intensities [PM-(MM₁+MM₂+MM₃)/3]. As expected are the specific intensities at 30 minutes incubation time up to 10 x weaker, but all perfect match positions can still be identified unequivocally. This test was carried out under a cover glass, so the hybridization was in this case a diffusion limited process. The same test done under agitation of the hybridization solution as during hybridization in a hybridization station yields higher intensities, so that the hybridization can be reduced to less than 30 minutes. This reduces the total assay time to < 3,5 hours.

### 9. Results

The array for genotyping of TEM beta lactamase as lined out above has shown a reproducible and sensitive identification of various genes of ESBL type beta lactamase. With an assay time of less than 3.5 hours this method is a very fast detection method for ESBL and/or IRT TEM beta lactamase and should be a valuable tool to monitor the spread of these resistance genes within the clinical setting.

## Claims

1. A method for detecting the presence or absence of a beta-lactam resistant micro-organism in a biological sample and simultaneously determining the genotype of the beta-lactam resistance, said method comprising the steps of:
(i) obtaining a biological sample;
(ii) optionally isolating DNA contained in the sample;
(iii) contacting the DNA of the sample with a micro-array, harboring on predetermined locations thereon different sets of capture probes, under conditions allowing hybridization of complementary strands,
(a) wherein each representative of a set of capture probes comprises the sequence R¹-(X)-R², which sequence represents a selected part of the sequence of a beta-lactamase gene, wherein X represents a nucleotide triplet and its permutations, and wherein R¹ and R² each have a length of from about 3 to 20 nucleotides,
(b) wherein the different sets of capture probes are selected such that an adjacent set starts at a given position 3n of nucleotides down-stream from the first set of capture probes, wherein n is an integer of 1 to 10, so that the nucleotide sequence of the beta-lactamase gene is covered over a desired range, and
(iv) determining whether and hybridization occurs and at which position on the array,
wherein the occurrence and location of a hybridization is indicative of the presence of a beta-lactam resistant micro-organism and also indicative of its specific resistance.

2. The method according to claim 1, wherein the DNA is isolated from the biological sample prior to contacting it with the array.

3. The method according to claim 1 or 2, wherein the DNA contained in the sample is amplified by means of one or more primer(s).

4. The method according to claim 3, wherein the amplified DNA is fragmented prior to the contacting step.

5. The method according to claim 5; wherein the sequence R₁-X-R₂ is derived from a beta-lactamase of the micro-organisms belonging to the group of *E.coli, Enterobacteiacae, Pseudomonas, Haemophilus,* and *Neisseria, Enterobacter, Klebsiella, Enterobacteiacae (Enterobacter aerogenes, Morganella morganii, Proteus mirabilis Proteus rettgeri, Proteus* spp., *Providencia* spp. *Salmonella* spp). *Klebsiella (K. pneumoniae, K ozaena, K. oxytoca), Capnocytophaga ochrace, Citrobacter spp.*, *Serratia marcescens, Shigella dysenteriae, Burkholderia cepacia.*

6. The method according to any of the preceding claims, wherein the capture probes are selected, so that the triplet to be permutated covers known location of SNP's in the beta-lactamase gene.

7. The method according to claim 5 or 6, wherein the beta-lactamase is a serin- or a zink-beta-lactamase.

8. The method according claim 5 to 7, wherein the beta-lactamase is selected from the group comprising the TEM beta-lactamase, SHV beta-lactamase, OXA beta-lactamase, or a beta-lactamase exhibiting an Extended Spectrum (ESBL) phenotype or an Inhibitor Resistant TEM (IRT) phenotype.

9. The method according to claim 8, wherein the sequence derived from a beta-lactamase gene is as shown in table I.

10. The method according to any of the claims 4 to 7, wherein the target DNA is fragmented to fragments having a size of about 15 to about 50 bp.

11. The method according to any of the preceding claims 1, wherein the DNA obtained from the sample, or amplified or fragmented is labeled prior to contacting it with the capture probes.

12. The method according to claim 1 to 8, wherein the labeling reaction is carried out on the array.

13. The method according to any of the claims 9 or 10, wherein the label is selected from the group of fluorescence label, colorimetric label, radioactive label, and electrically, electrochemically and/or enzymatically detectable label.

14. A kit for detecting the presence or absence of a beta-lactam resistant micro-organism in a biological sample and simultaneously determining the genotype of the beta-lactam resistance, which comprises:
(i) a micro-array, harboring on predetermined locations thereon different sets of capture probes,
(a) wherein each representative of a set of capture probes comprises the sequence R¹-(X)-R², which sequence represents a selected part of the sequence of a beta-lactamase gene, wherein X represents a nucleotide triplet and its permutations, and wherein R¹ and R² each have a length of from about 5 to 20 nucleotides,
(b) wherein the different sets of capture probes are selected such that an adjacent set starts at a given position 3n of nucleotides down-stream from the first set of capture probes, wherein n is an integer of 1 to 10, so that the nucleotide sequence of the beta-lactamase gene is covered over a desired range, and
(ii) buffers and reagents for performing the method.
